# EUROPEAN PATENT APPLICATION

(11) **EP 4 722 380 A1**
(43) Date of publication of application: **08.04.2026**
(21) Application number: 24810494.5
(22) Date of filing: 24.05.2024
(51) Int. Cl.: C12Q 1/6806, C12Q 1/6869, C40B 50/06

(54) **USE OF FORMALDEHYDE HEAVY CROSSLINKING IN CUT&TAG LIBRARY CONSTRUCTION**

(30) Priority: 24.05.2023 CN 202310592949
(71) Applicant: Novoprotein Scientific (Shanghai) Inc., Shanghai 201203 (CN); Novoprotein Scientific Inc., Suzhou, Jiangsu 215200 (CN)
(72) Inventor: LI, Ke, Shanghai 201203 (CN); SHI, Yingdong, Shanghai 201203 (CN)
(74) Representative: Lavoix
(86) International application number: PCT/CN2024/095287
(87) International publication number: WO 2024/240259

(57) **Abstract**

Provided a methed for formaldehyde heavy cross-linking in CTR&Tag library construction and the use thereof. The method involves low cell demands, a high signal-to-noise ratio and relatively simple operation, and achieves the advantages of formaldehyde heavy cross-linking to immobilize cells in ChIP experiments. The method has remarkable advantages in exploring protein-DNA weak interactions.

## Description

### Technical field

The present invention relates to the field of biotechnology, and in particular to the application of formaldehyde heavy cross-linking in library construction using the CUT&Tag technology.

### Background

During normal embryonic development, a fertilized egg carrying identical genetic material differentiates into cells with varying morphologies, ultimately forming a complete organism with distinct tissues and organs. Studies have shown that this differentiation process, which does not involve changes in genetic material, is achieved through epigenetic regulation. Epigenetic regulation typically includes gene regulation and chromatin regulation, these regulation processes are closely related to protein-DNA interactions. Disruption of epigenetically controlled gene expression patterns can lead to various diseases, including autoimmune diseases and cancer. Therefore, studying protein-DNA interactions is crucial for understanding the occurrence of disease.

Currently, the main methods for studying protein-DNA interactions include chromatin immunoprecipitation (ChIP), CUT&RUN, and CUT&Tag (Cleavage Under Targets and Tagmentation) techniques. CUT&Tag technology uses cells or cell nuclei for experiments, bypassing the need for cross-linking and immobilizing cells (or using light cross-linking with 0.1-0.2% formaldehyde for 2-3 minutes), instead achieving targeted disruption through protein A/protein G-Tn5 transposase. It features low cell requirements (10,000-100,000 cells), short processing time (8-10 hours), low background noise, and simple experimental procedures.

However, in extensive practice, researchers have discovered that CUT&Tag technology has limitations. For the detection of interactions between DNA-binding proteins, which bind dynamically to DNA, the non-cross-linking or light cross-linking (0.1% to 0.2% formaldehyde) methods used in CUT&Tag technology are difficult to truly reflect the objective conditions of experimental samples. Furthermore, there are also issues with weak signal intensity at binding sites and high background.

In summary, CUT&Tag technology faces enormous challenges. How to make CUT&Tag applicable to most protein-DNA interactions while maintaining the simplicity and speed of CUT&Tag technology is an urgent problem to be solved.

### Summary of the invention

The purpose of the present invention is to provide an application of formaldehyde cross-linking in CUT&Tag technology library construction.

The purpose of the present invention is also to provide a library construction method for target protein immobilization and DNA binding pattern identification.

The purpose of the present invention is also to provide a library construction method for identifying DNA binding patterns of DNA binding proteins that have weak DNA binding affinity or highly dynamic DNA binding.

In the first aspect of the present invention, it provides a method for constructing a nucleic acid library, comprising:
(S1) providing a cell sample;
(S2) immobilizing the cell sample with a cross-linking agent having a final concentration of C1 (v/v) for a time period of t1 (min), where the immobilization integral I1 = C1 * t1, and I1 ≥ 2.5, to obtain a non-permeabilized cell sample;
(S3) permeabilizing the non-permeabilized cell sample to obtain a permeabilized cell sample;
(S4) washing the permeabilized cell sample to obtain a washed cell mixture;
(S5) mixing the cell mixture with pretreated magnetic beads to obtain a cell-magnetic bead complex;
(S6) treating the cell-magnetic bead conjugate with a primary antibody to obtain a primary antibody-cell-magnetic bead complex;
(S7) treating the primary antibody-cell-magnetic bead complex with a secondary antibody to obtain a secondary antibody-primary antibody-cell-magnetic bead complex;
(S8) treating the secondary antibody-primary antibody-cell-magnetic bead complex with a transposase to perform a fragmentation reaction to form DNA fragments;
(S9) terminating the reaction and decross-linking to obtain a reaction-terminated product; and
(S10) separating, purifying, and amplifying the DNA fragments in the reaction-terminated product to prepare a nucleic acid library.

In another preferred embodiment, in (S1), the cells comprise animal cells, preferably mammalian cells, more preferably hamster cells, human cells or mouse cells.

In another preferred embodiment, in (S1), the cell density in the cell sample is 1×10³ ~5x10⁶ cells/mL.

In another preferred embodiment, in (S2), C1 is 0.5% to 5% (v/v); preferably 1% to 3% (v/v).

In another preferred embodiment, in (S2), t1 is 5 to 60 min; preferably 10 to 20 min.

In another preferred embodiment, in (S2), I1 is 2.5-300; preferably I1≥10.

In another preferred embodiment, in (S2), the immobilizing is a complete immobilizing, meaning that all or substantially all cells are cross-linked and immobilized; wherein "all or substantially all" means that ≥85%; preferably ≥ 90%; preferably ≥95%; more preferably ≥98%; and optimally ≥99% of the cells are cross-linked and immobilized.

In another preferred embodiment, in (S2), the cross-linking agent includes, but is not limited to, formaldehyde, EGS, and DSG, preferably formaldehyde.

In another preferred embodiment, in (S2), the immobilizing includes:
(S2.1) according to a cell density of 3×10³ to 3×10⁶ cells/mL, adding a corresponding volume of a 0.5% to 5% formaldehyde cross-linking agent solution to the cell sample of (S1), and incubating at room temperature for 5 to 60 min;
(S2.2) terminating the reaction with glycine at a final concentration of 125 to 1000 mM for a termination time of 5 to 15 min.

In another preferred embodiment, in (S2), the permeabilization buffer comprises PB, Tris, or HEPES buffer.

In another preferred embodiment, in (S2), the permeabilization agent is selected from one or more of the groups consisting of: Tween20, SDS, NP40 (or IGEPAL CA-630), Digtonin, CHAPS, sodium deoxycholate, or a combination thereof; preferably Tween20, SDS or NP40 (or IGEPAL CA-630).

In another preferred embodiment, in (S3), the permeabilization buffer comprises one or more selected from the group consisting of:
0.1% to 0.5% (v/v), preferably 0.2% (v/v) NP40,
0.1% to 0.5% (v/v), preferably 0.1% (v/v) SDS,
0.1% to 0.5% (v/v), preferably 0.1% (v/v) Tween20,
0.01% to 0.2% (v/v), preferably 0.05% (v/v) Digitonin,
0.1% to 0.5% (v/v), preferably 0.1% (v/v) TritonX-100,
0.1% to 0.5% (v/v), preferably 0.1% (v/v) CHAPS.

In another preferred embodiment, in (S3), the permeabilization treatment comprises: treating the immobilized cell sample with a permeabilization buffer containing a permeabilization agent at a concentration C2 (v/v) for a time period of t2 (min) to obtain the permeabilized cell sample.

In another preferred embodiment, in (S3), C2 is 0.005% to 5%;preferably 0.01% to 2%.

In another preferred embodiment, in (S3), t2 is 5 to 60 min.

In another preferred embodiment, in (S3), the cell density for the permeabilization treatment is 3×10³ to 3×10⁶ cells/mL.

In another preferred embodiment, in (S3), the temperature for the permeabilization treatment is 45 to 68°C.

In another preferred embodiment, in (S3), the pH value of the permeabilization buffer is 7.0-7.8; preferably 7.2-7.6.

In another preferred embodiment, in (S3), the permeabilization buffer contains 100 to 200 mM NaCl or other salts with equivalent conductivity.

In another preferred embodiment, in (S3), the permeabilization treatment comprises: treating the immobilized cell sample with a permeabilization buffer, and incubating the cell sample in a temperature-controlled instrument at 55 to 65 °C for 5 to 20 min.

In another preferred embodiment, in (S4), the washing comprises: adding cell washing buffer, incubating for 20 to 50 minutes, and then centrifuging to remove the supernatant.

In another preferred embodiment, in (S4), the cell washing buffer is a buffer containing 0.01% to 0.1% (v/v) permeabilization agent.

In another preferred embodiment, in (S4), the cell washing buffer comprises one or more permeabilization agents selected from the group consisting of:
0.1% to 0.5% (v/v), preferably 0.15% (v/v) Tween20,
0.1% to 0.5% (v/v), preferably 0.15% (v/v) NP40,
0.01% to 0.2% (v/v), preferably 0.01% (v/v) Digitonin,
0.1% to 0.5% (v/v), preferably 0.1% (v/v) TritonX-100.

In another preferred embodiment, in (S5), the magnetic beads are ConA magnetic beads.

In another preferred embodiment, in (S6), the incubation time with the primary antibody is 1 to 16 h.

In another preferred embodiment, in (S8), the transposase is selected from: pA-Tn5, pG-Tn5, pA-pG-Tn5; preferably pA/G-Tn5 (protein A-Tn5).

In another preferred embodiment, in (S8), the incubation interruption time of the transposase is 0.5 to 3 hours.

In another preferred embodiment, in (S9), the terminating the reaction and decross-linking comprises: adding a decross-linking agent for incubation.

In another preferred embodiment, in (S9), the decross-linking reagent includes: 2-10 µL of 0.5 M EDTA; 1-8 µL of 10% SDS; 0.2-5 µL of 20 mg/mL proteinase K.

In another preferred embodiment, in (S9), the temperature for the decross-linking is 50 to 60°C; and the time for the decross-linking is 0.5 to 12 hours.

In the second aspect of the present invention, it provides a kit for constructing a nucleic acid library using CUT&Tag technology, comprising:
(1) a cross-linking agent solution;
(2) a permeabilization buffer;
(3) a cell washing buffer;
(4) a fragmentation reaction reagent; and
(5) a decross-linking reagent.

In another preferred embodiment, the cross-linking agent includes but is not limited to, formaldehyde, EGS, and DSG, preferably formaldehyde.

In another preferred embodiment, the concentration C1% of the cross-linking agent is 0.5% to 5%, preferably 1% to 3%.

In another preferred embodiment, the permeabilization buffer comprises PB, Tris, or HEPES buffer.

In another preferred embodiment, the pH value of the permeabilization buffer is 7.0-7.8, preferably 7.2-7.6.

In another preferred embodiment, the permeabilization buffer contains 50-150 mM NaCl or other salts with equivalent conductivity.

In another preferred embodiment, the permeabilization buffer further contains a cell protein protectant, which is 1 to 100 mM Glycine, 0.1%-2% (v/v) BSA, or other analogues.

In another preferred embodiment, the permeabilization buffer contains a permeabilization agent selected from the group consisting of: Tween20, SDS, NP40, Digtonin, CHAPS, sodium deoxycholate, or a combination thereof; preferably Tween20, SDS or NP40.

In another preferred embodiment, the permeabilization buffer comprises one or more selected from the group consisting of:
0.1% to 0.5% (v/v), preferably 0.2% (v/v) NP40,
0.1% to 0.5% (v/v), preferably 0.1% (v/v) SDS,
0.1% to 0.5% (v/v), preferably 0.1% (v/v) Tween20,
0.01% to 0.2% (v/v), preferably 0.05% (v/v) Digitonin,
0.1% to 0.5% (v/v), preferably 0.1% (v/v) TritonX-100,
0.1% to 0.5% (v/v), preferably 0.1% (v/v) CHAPS.

In another preferred embodiment, the concentration C2 of the permeabilization agent is 0.005% to 5%, preferably 0.01% to 2%.

In another preferred embodiment, the cell washing buffer is a buffer containing 0.01% to 0.1% permeabilization agent.

In another preferred embodiment, the permeabilization agent contained in the cell washing buffer is selected from the group consisting of: Tween20, TritonX100, SDS, NP40, Digtonin, or a combination thereof.

In another preferred embodiment, the pH value of the cell washing buffer is 7.2 to 7.6.

In another preferred embodiment, the cell washing buffer contains 100 to 150 mM NaCl or other salts with equivalent conductivity.

In another preferred embodiment, the fragmentation reaction is performed by a transposase, and the transposase is selected from the group consisting of: pA/G-Tn5, protein A-Tn5, protein G-Tn5, protein A-protein G-Tn5, or a combination thereof.

In another preferred embodiment, the fragmentation reaction reagent includes but is not limited to transposase, magnetic bead, primary antibody, and secondary antibody.

In another preferred embodiment, the kit further comprises a decross-linking reagent, a reaction termination reagent, or a reagent for PCR amplification.

In another preferred embodiment, the decross-linking reagent includes: 2 to 10 µL 0.5 M EDTA; 1 to 8 µL 10% SDS; and 0.2 to 5 µL 20 mg/mL proteinase K.

In the third aspect of the present invention, it provides a method for measuring protein-chromatin interactions, comprising:
(S1) constructing a nucleic acid library using the method of claim 1; and
(S2) sequencing the nucleic acid library to generate a plurality of sequencing reads for measuring protein-chromatin interactions.

In another preferred embodiment, the protein comprises a DNA-binding protein that has weak DNA binding affinity or highly dynamic DNA binding.

It should be understood that within the scope of the present invention, the various technical features of the present invention above and the various technical features specifically described hereinafter (as in the examples) may be combined with each other to constitute a new or preferred technical solution. And it needs not be described one by one, due to space limitations.

### DESCRIPTION OF THE DRAWINGS

Figure 1 shows a schematic diagram of the formaldehyde light cross-linking process of the ChiTag of the present invention and the classic CUT&Tag/CUT&Tag.
Figure 2 shows the electrophoresis pattern of failed library construction using the classic CUT&Tag process after formaldehyde heavy cross-linking.
Figure 3 shows a comparison of electrophoresis patterns of libraries constructed using the CUT&Tag and ChiTag processes after formaldehyde heavy cross-linking.
Figure 4 shows the electrophoretic patterns of different antibodies in library construction using the CUT&Tag (classic non-cross-linked method) and ChiTag processes.
Figure 5 shows a comparison of the signal-to-noise ratio maps (partially extracted) of different antibodies (CTCF, GR, Pol II, H3K4me2) in sequencing of libraries constructed using the CUT&Tag and ChiTag processes.
Figure 6 shows the peak number statistics of different antibodies (CTCF, GR, Pol II, H3K4me2) in sequencing of library constructed using the CUT&Tag and ChiTag processes to build libraries for sequencing.

### Detailed description

Through extensive and deep research, the inventors unexpectedly discovered, for the first time, that efficient and rapid construction of nucleic acid libraries can be achieved by utilizing heavy cross-linking (e.g., using 0.5% to 5% formaldehyde for 5 to 60 minutes) for cell immobilization, followed by cell permeabilization treatment, in combination with CUT&Tag technology and subsequent decross-linking. In the present invention, non-permeabilized cell samples obtained after thorough cross-linking fail to produce fragmentation bands when processed via the classical CUT&Tag method without the cell permeabilization step. The ChiTag method of the present invention requires a lower number of cells (below 100,000), yields high sequencing signal-to-noise ratio, involves relatively simple operations, and simultaneously leverages the advantages of formaldehyde heavy cross-linking immobilization from ChIP experiments. The library yield constructed by this method can be increased by 300 to 1000 times compared to the classical CUT&Tag method. Notably, the ChiTag method demonstrates significant advantages in investigating weak protein-DNA interactions. The present invention has been completed based on these findings.

### Terms

Unless defined otherwise, all technical and scientific terms used herein have the same meaning as commonly understood by one of ordinary skill in the art to which this invention belongs.

The term "about" can refer to a value or composition that is within an acceptable error range for the particular value or composition as determined by one of ordinary skill in the art, which will depend in part on how the value or composition is measured or determined.

As used herein, the terms "comprising" or "including" may be open, semi-closed, or closed. In other words, the terms also include "consisting essentially of" or "consisting of."

### Methods for studying protein-DNA interactions

Currently, there are three main methods for studying protein-DNA interactions:
1. Chromatin immunoprecipitation (ChIP): The process is as follows: cross-linking and immobilizing cells with 1% to 5% formaldehyde → ultrasonically disrupting chromatin → immunoprecipitation → DNA fragment library construction and sequencing. This method has disadvantages such as large material requirements (1 million to 10 million cells), long processing time (3 to 5 days), high background noise, high antibody consumption, and poor experimental reproducibility. In addition, the method is cumbersome and difficult to operate, further limiting its use.
2. CUT&RUN, a method for targeted disruption and library construction using a target-specific primary antibody combined with protein A/protein G-micrococcal enzyme (pA/G-MNase). This method does not require formaldehyde cross-linking, is less time-consuming (1-2 days), requires a low cell requirement (100,000), and is significantly less difficult to perform than ChIP.
3. CUT&Tag technology: This technology uses cells or cell nuclei for experiments, without the need for cross-linking and immobilizing cells (or using 0.1-0.2% formaldehyde for 2-3 minutes of light cross-linking). Targeted fragmentation is achieved by binding the protein A/protein G-Tn5 transposase to the antibody. A high-salt buffer (no less than 300mM NaCl) is used to inhibit the cleavage efficiency of the transposase, reducing the generation of random fragments caused by nonspecific binding. This method has the advantages of low cell requirement (10,000-100,000 cells), short time consumption (8-10 hours), lower background noise, and simple experiments.

As used herein, " formaldehyde heavy cross-linking" typically refers to a cross-linking reaction using 1% to 5% formaldehyde for 10 to 60 minutes.

As used herein, " formaldehyde light cross-linking" typically refers to a cross-linking reaction using 0.1% to 0.2% formaldehyde for 2 to 3 minutes.

As used herein, "CUT&Tag light cross-linking," "CUT&Tag formaldehyde light cross-linking," or "classic CUT&Tag light cross-linking assay" are used interchangeably and generally refer to an experiment procedure in which cells are treated with formaldehyde light cross-linking followed by a classic CUT&T assay.

In conventional cell immobilization experiments using formaldehyde cross-linking, the commonly used formaldehyde concentration is 1% to 5% for 10 to 30 minutes. Under these conditions, cells are generally considered to be sufficiently immobilized, and the experimental results are widely regarded as reliable. Conventional ChIP experiments are commonly performed within this cross-linking range.

### The ChiTag method of the present invention

As used herein, "the ChiTag method of the present invention", "the ChiTag process of the present invention", "the method of combining formaldehyde heavy cross-linking and CUT&Tag of the present invention", and "the method of formaldehyde heavy cross-linking combined with CUT&Tag of the present invention" can be used interchangeably and all refer to the method of the first aspect of the present invention, specifically, a method of immobilizing cells by formaldehyde heavy cross-linking, permeabilizing the cells, and then combining with CUT&Tag technology.

It should be understood that the formaldehyde heavy cross-linking or cell immobilization treatment in the present invention preferably allows the cells to be fully cross-linked or fully immobilized. Preferably, 1% to 5% formaldehyde is used to heavy cross-link and immobilize the cells so that the binding of DNA binding proteins to chromosomes is immobilized. During the experiment, the DNA binding protein is linked to the DNA through a covalent bond, and the relative position is immobilized, which can more realistically reflect the actual situation of the sample at a specific time. Preferably, the cross-linking agent used for the immobilization treatment of cells of the present invention adopts a 0.5% to 5% formaldehyde solution, and the cross-linking time is 5 to 60 minutes; the cross-linking agent is not limited to formaldehyde, and includes other cross-linking agents with the same immobilization effect such as EGS and DSG; the cross-linking agent for cell immobilization in the technical process is not limited to one, and can be two or more cross-linking agents used in combination.

Specifically, the ChiTag method of the present invention, i.e., the technical process of combining formaldehyde heavy cross-linking with CUT&Tag, comprises the following steps:
Cell immobilization → termination of immobilization → cell permeabilization → cell washing → cell binding to magnetic beads → antibody incubation → pA/G-Tn5 incubation and fragmentation reaction → reaction termination and decross-linking → DNA extraction → library preparation.

The cell density of the cross-linking reaction system in the technical process is 3×10³ to 3×10⁶ cells/mL, and immobilization is terminated using Glycine at a final concentration of 125 to 1000 mM;
The buffer used for cell permeabilization in the technical process is a buffer containing 0.01% to 2% cell permeabilization agent.

The buffer is PB, Tris, HEPES, etc.; the cell membrane permeabilizer in the buffer includes, but is not limited to, one, two or a combination of multiple agents from Tween20, SDS, NP40, Digtonin, CHAPS, and sodium deoxycholate; the pH value of the buffer is 7.0-7.8; the buffer contains 50-150 mM NaCl, or other salts with equivalent conductivity; the buffer contains 1-100 mM Glycine, 0.1%-2% BSA, or other similar agents as cell protein protectants.

The cell density for cell permeabilization in the technical process is 3×10³ to 3×10⁶ cells/mL.

The time for cell permeabilization in the technical process is 5 to 60 min; the temperature for permeabilization is 45 to 68°C.

The cell washing buffer in the technical process is a buffer containing 0.01% to 0.1% cell membrane permeabilization agent.

The buffer comprises PB, Tris, HEPES, etc.; the permeabilization agent in the buffer comprises but is not limited to one, two, or a combination of multiple agents from Tween20, TritonX100, SDS, NP40 and Digtonin; the pH value of the buffer is 7.2-7.6; the buffer contains 150 mM NaCl or other salts with equivalent conductivity.

The magnetic beads in the process are ConA magnetic beads.

The primary antibody incubation time in the technical process is 1 to 16 h.

The transposase in the technical process is pA/G-Tn5, which can be any one of protein A-Tn5, protein G-Tn5, and protein A-protein G-Tn5.

The time for the pA/G-Tn5 incubation interruption in the technical process is 0.5 to 3 h.

The temperature for the decross-linking in the technical process is 50 to 60°C; the time for the decross-linking in the technical process is 0.5 to 12 h.

### The main advantages of the present invention include

1. In experiment of cells immobilization using formaldehyde heavy cross-linking, libraries constructed via the ChiTag process exhibited a 300 to 1000-fold increase in yield compared to those constructed using the CUT&Tag light cross-linking process.
2. Formaldehyde heavy cross-linking for cells immobilization while simultaneously performing ChiTag assay, compared to the classic CUT&Tag non-cross-linked cells assay, it was found that the sequencing performance for transcription factors and dynamic DNA-binding proteins was superior with the ChiTag method. Using the same number of cells, the same antibody for library construction, and the same sequencing depth, the ChiTag process produced a higher number of peaks and a higher signal-to-noise ratio than the traditional CUT&Tag process. For some transcription factors, no peaks were detected using the CUT&Tag process, whereas the ChiTag process yielded clear enrichment signals.

The present invention will be further described below in conjunction with specific examples. It should be understood that these examples are intended to illustrate the present invention only and are not intended to limit the scope of the present invention. The experimental methods in the following examples, for which detailed conditions are not specified, were generally performed under conventional conditions such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

In this example, concanavalin A magnetic beads (ConA beads, N251), CUT&Tag kit (CUT&Tag 3.0, N259), DNA extraction magnetic beads (Tagment DNA Exract Beads, N245, NovoNGSDNA Clean Beads, N240), and secondary antibodies (Goat Anti-Rabbit IgG H&L, N269, Goat Anti-Mouse IgG H&L, N270) from Suzhou Jinan Protein Technology Co., Ltd. were used. The primary antibodies used in this example were purchased from EMD Millipore (H3K4me2, 07-030, CTCF, 07-729); Cell Signaling (Pol II, 2629S); Santa Cruz Biotechnology (GR, sc-393232), and ACTIVE MOTIF (H3K4me3, 39159). The cells used in the present invention are human K562 cells.

**Table 1 Primer sequences**

| **Primer Name** | **Primer sequences(5'-3')** | **SEQ ID NO.** |
|---|---|---|
| N501 | AATGATACGGCGACCACCGAGATCTACACTAGATCGCTCGTCGGCAGCGTCA | 1 |
| N502 | AATGATACGGCGACCACCGAGATCTACACCTCTCTATTCGTCGGCAGCGTCA | 2 |
| N503 | AATGATACGGCGACCACCGAGATCTACACTATCCTCTTCGTCGGCAGCGTCA | 3 |
| N504 | AATGATACGGCGACCACCGAGATCTACACAGAGTAGATCGTCGGCAGCGTCA- | 4 |
| N505 | AATGATACGGCGACCACCGAGATCTACACGTAAGGAGTCGTCGGCAGCGTCA- | 5 |
| N701 | CAAGCAGAAGACGGCATACGAGATTCGCCTTAGTCTCGTGGGCTCGG | 6 |
| N702 | CAAGCAGAAGACGGCATACGAGATCTAGTACGGTCTCGTGGGCTCGG | 7 |
| N703 | CAAGCAGAAGACGGCATACGAGATTTCTGCCTGTCTCGTGGGCTCGG | 8 |
| N704 | CAAGCAGAAGACGGCATACGAGATGCTCAGGAGTCTCGTGGGCTCGG | 9 |
| N705 | CAAGCAGAAGACGGCATACGAGATAGGAGTCCGTCTCGTGGGCTCGG | 10 |
| P5 | AATGATACGGCGACCACCGAGATCTACAC | 11 |
| P7 | CAAGCAGAAGACGGCATACGAGAT | 12 |

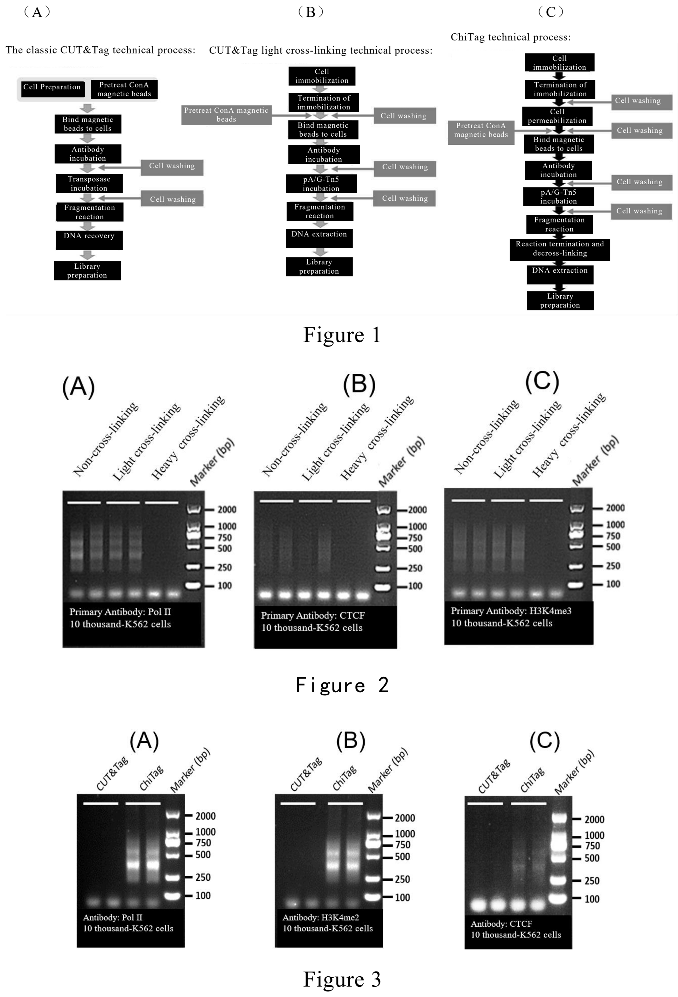

The classic CUT&Tag experimental process, the CUT&Tag light cross-linking experimental process, and the ChiTag experimental process for heavy cross-linked immobilized cells are shown in Figure 1.

### S1 the classic CUT&Tag process is as follows (using the CUT&Tag kit, the experimental process is shown in Panel A of Figure 1):

1. Cell Preparation
   1) Collect and count fresh cells at room temperature. Place the required number of cells into a new 1.5 mL centrifuge tube, and centrifuge at 600 g for 5 min at room temperature and carefully remove the supernatant.
   2) Add 1 mL of Wash Buffer to the cell pellet from the previous step, mix gently by pipetting, and centrifuge at 600 g for 5 min at room temperature and discard the supernatant.
   3) Add 90 µL Wash Buffer to the cell pellet from the previous step and resuspend the cells.
2. Bind ConA magnetic beads to cells
   Add 10 µL of pretreated ConA magnetic beads to the 90 µL sample from the previous step, mix gently by pipetting, and place on a rotating mixer at room temperature for 10 min.
3.Bind ConA magnetic beads-cells to primary antibody
   1) Dilute the primary antibody with Primary Antibody Buffer. Each experimental sample requires 50 µL of primary antibody diluent. The primary antibody dilution ratio is based on the instructions. Place the prepared primary antibody diluent on ice until ready for use.
   2) Place the centrifuge tube containing ConA magnetic beads-cells that have been prepared in step 2 on a magnetic rack for 2 minutes. After the magnetic beads are completely separated from the liquid, carefully remove the supernatant. Remove the centrifuge tube, add 50 µL of primary antibody diluent, and incubate at room temperature for 2 hours or at 4°C overnight.
4.Bind ConA magnetic beads-cells to secondary antibody and wash
   1) Remove the centrifuge tube after primary antibody incubation and place it on a magnetic rack for 2 min. After the magnetic beads are completely separated from the liquid, carefully remove the supernatant.
   2) Remove the centrifuge tube from the magnetic rack, add 100 µL of secondary antibody diluent, resuspend the ConA magnetic beads-cells, gently pipette to mix, and incubate at room temperature for 1 h;
   3) After the secondary antibody incubation is complete, place the centrifuge tube on a magnetic rack for 2 minutes and carefully remove the supernatant.
   4) Remove the centrifuge tube from the magnetic rack, add 200 µL Antibody Buffer, resuspend the ConA magnetic beads-cells, gently pipette to mix, and let stand at room temperature for 3 min;
   5) Repeat steps 4-3) and 4) once.
5. Transposome binding and washing
   1) Place the sample after the previous step on a magnetic rack and let stand for 2 min. After the magnetic beads are completely separated from the liquid, carefully remove the supernatant.
   2) Remove the centrifuge tube from the magnetic rack, add 90 µL of ChiTag transposome diluent, gently pipette to resuspend the cells, and incubate the mixed sample at room temperature for 1 h.
   3) Place the centrifuge tube for incubating transposon on a magnetic rack and let stand for 2 min. After the magnetic beads are completely separated from the liquid, carefully remove the supernatant.
   4) Remove the centrifuge tubes from the magnetic rack and add 200 µL ChiTag Buffer to each tube of sample. Mix gently by pipetting to resuspend all ConA magnetic beads-cells and let stand at room temperature for 3 min.
   5) Repeat steps 5-3) and 4) once.
6. Fragmentation reaction
   1) Place the sample after the previous step on a magnetic rack and let stand for 2 min. After the magnetic beads are completely separated from the liquid, carefully remove the supernatant;
   2) Remove the centrifuge tube from the magnetic rack, add 40 µL Tagmentation Buffer, gently pipette to mix the cells, and place in an incubator at 37°C for 1 h. Obtain 5× Stop Buffer and thaw at room temperature for later use.
   3) Vortex mix the 5× Stop Buffer. Add 10 µL of 5× Stop Buffer to the incubated sample, pipette or vortex mix thoroughly, and incubate in a metal bath or water bath at 55°C for 10 min to terminate the fragmentation reaction.

### S2 the classic CUT&Tag light cross-linking process is as follows (using the CUT&Tag kit, the experimental process is shown in Panel B of Figure 1):

1. Cell Preparation
   1) Collect and count the cells. Place the required cells into a new 1.5 mL centrifuge tube and centrifuge at 600 g for 5 min. Carefully remove the supernatant.
   2) Add 1 mL of PBS to the cell pellet from the previous step to resuspend the cells, centrifuge at 600 g for 5 min at room temperature, and discard the supernatant.
2. Formaldehyde cross-linking and termination
   1) Prepare formaldehyde solution (in PBS) with a final concentration of 0.1% in advance;
   2) Perform formaldehyde cross-linking at a cell density of 3×10⁶ cells/mL, incubating at room temperature for 2 min;
   3) Add 2.5 M glycine to a final concentration of 125 mM and incubate at room temperature for 5 min to neutralize the reaction;
   4) Centrifuge at 600g for 5 min and resuspend the cells in 90 µL Wash Buffer.
3.Bind ConA magnetic beads to cells
   Add 10 µL of pretreated ConA magnetic beads to the 90 µL sample from the previous step, mix gently by pipetting, and place on a rotating mixer at room temperature for 10 min.
4.Bind ConA magnetic beads-cells to primary antibody
   1) Dilute the primary antibody with Primary Antibody Buffer. Each experimental sample requires 50 µL of primary antibody diluent. The primary antibody dilution ratio is based on the instructions. Place the prepared primary antibody diluent on ice until ready for use.
   2) Place the centrifuge tube containing ConA magnetic beads-cells that have been prepared in step 2 on a magnetic rack for 2 minutes. After the magnetic beads are completely separated from the liquid, carefully remove the supernatant. Remove the centrifuge tube, add 50 µL of primary antibody diluent, and incubate at room temperature for 2 h or at 4°C overnight.
5.Bind ConA magnetic beads-cells tosecondary antibody and wash
   1) Remove the centrifuge tube after primary antibody incubation and place it on a magnetic rack for 2 min. After the magnetic beads are completely separated from the liquid, carefully remove the supernatant;
   2) Remove the centrifuge tube from the magnetic rack, add 100 µL of secondary antibody diluent, resuspend the ConA magnetic beads-cells, gently pipette to mix, and incubate at room temperature for 1 h;
   3) After the secondary antibody incubation is complete, place the centrifuge tube on a magnetic rack for 2 min and carefully remove the supernatant;
   4) Remove the centrifuge tube from the magnetic rack, add 200 µL Antibody Buffer, resuspend the ConA magnetic beads-cells, gently pipette to mix, and let stand at room temperature for 3 min;
   5) Repeat steps 4-3) and 4-4) once.
6. Transposome binding and washing
   1) Place the sample after the previous step on a magnetic rack and let stand for 2 min. After the magnetic beads are completely separated from the liquid, carefully remove the supernatant;
   2) Remove the centrifuge tube from the magnetic rack, add 90 µL of ChiTag transposome diluent, gently pipette to resuspend the cells, and incubate the mixed sample at room temperature for 1 h;
   3) Place the centrifuge tube for incubating transposon on a magnetic rack and let stand for 2 minutes. After the magnetic beads are completely separated from the liquid, carefully remove the supernatant.
   4) Remove the centrifuge tubes from the magnetic rack and add 200 µL ChiTag Buffer to each tube of sample. Mix gently by pipetting to resuspend all ConA magnetic beads-cells. Let stand at room temperature for 3 min.
   5) Repeat steps 5-3) and 5-4) once.
7. Fragmentation reaction
   1) Place the sample after the previous step on a magnetic rack and let stand for 2 minutes. After the magnetic beads are completely separated from the liquid, carefully remove the supernatant;
   2) Remove the centrifuge tube from the magnetic rack, add 40 µL Tagmentation Buffer, gently pipette to mix the cells, and place in an incubator at 37°C for 1 h. Obtain 5× Stop Buffer and thaw at room temperature for later use;
   3) Vortex mix the 5× Stop Buffer. Add 10 µL of 5× Stop Buffer to the incubated sample, pipette or vortex mix thoroughly, and incubate in a metal bath or water bath at 55°C for 10 min to terminate the fragmentation reaction.

### S3 the ChiTag experimental process is as follows (using the CUT&Tag kit, the experimental process is shown in Panel C of Figure 1):

1. Cell Preparation
   1) Collect and count fresh cells at room temperature, place the required number of cells into a new 1.5 mL centrifuge tube, centrifuge at 600 g for 5 min at room temperature and discard the supernatant.
   2) Add 1 mL of PBS and mix gently by pipetting, centrifuge at 600 g for 5 min at room temperature and discard the supernatant.
2. Formaldehyde cross-linking
   1) Prepare formaldehyde solution (formaldehyde-PBS) with a final concentration of 1% in advance;
   2) Based on a cell density of 3×10⁶ cells/mL, add the corresponding volume of 1% formaldehyde-PBS solution to the cell pellet from step 1-2), incubate at room temperature for 10 min.
3. Termination of cross-linking
   1) Add 2.5 M glycine stock solution to the formaldehyde cross-linking system to make the final glycine concentration in the reaction system 125 mM and let stand at room temperature for 5 min;
   2) Centrifuge at 1300 g for 5 min at room temperature and discard the supernatant;
   3) Add 1 mL of PBS to the cell pellet to resuspend the cells, centrifuge at 1300 g for 5 min at room temperature, and discard the supernatant;
   4) Repeat steps 3-3) once.
4. Cell Permeabilization
   Add 200 µL of permeabilization buffer (PBS, 0.2% NP40, 0.1% SDS, 0.1% Tween 20, 0.05% Digitonin, 0.1% Trinon X-100, 0.1% CHAPS, 50 mM Glycine) to the precipitate in step 3-4), and incubate in a 58°C metal bath for 10 min.
5. Washing
   1) Add 800 µL of cell washing buffer Wash Buffer (PBS, pH 7.4, 0.5 mM Spermidine, 0.15% Tween 20, 0.15% NP40, 0.01% Digitonin, 0.1% Triton X-100) to the EP tube from the previous step. Mix gently by pipetting, and incubate at 37°C for 30 min, then centrifuge at 600 g for 5 min at room temperature, and discard the supernatant;
   2) Add 90 µL Wash Buffer to the pellet from the previous step and resuspend the cells.
6.Bind ConA magnetic beads to cells
   Add 10 µL of pretreated ConA magnetic beads to 90 µL of sample, mix gently by pipetting, and incubate on a rotating mixer at room temperature for 10 min.
7.Bind ConA-cells to primary antibody
   1) Dilute the primary antibody with Primary Antibody Buffer, each experimental sample requires 50 µL of primary antibody diluent, the primary antibody dilution ratio is based on the instructions, and place the prepared primary antibody diluent on ice until ready for use;
   2) Place the ConA magnetic beads-cells incubated in step 1 on a magnetic rack for 2 minutes, after the magnetic beads are completely separated from the liquid, carefully remove the supernatant. Remove the EP tube from the magnetic rack, add 50 µL of pre-chilled primary antibody diluent, pipette to mix, and incubate at room temperature for 2 h or at 4°C overnight.
8.Bind ConA-cells to secondary antibody and wash
   1) Remove the EP tube after primary antibody incubation and place it on a magnetic rack for 2 min. After the magnetic beads are completely separated from the liquid, carefully remove the supernatant;
   2) Remove the EP tube from the magnetic rack, add 100 µL of secondary antibody diluent, resuspend the ConA magnetic beads-cells, gently pipette to mix, and incubate at room temperature for 1 h;
   3) Place the EP tube after secondary antibody incubation on a magnetic rack for 2 min. After the magnetic beads are completely separated from the liquid, carefully remove the supernatant.
   4) Remove the EP tube from the magnetic rack, add 200 µL Antibody Buffer, resuspend the ConA magnetic beads-cells, gently pipette to mix, and let stand at room temperature for 3 min;
   5) Repeat steps 3) and 4) once.
9.Incubate ConA- cells with pA/G-Tn5-MgCl₂
   1) Place the sample from the previous step on a magnetic rack and let stand for 2 min, after the magnetic beads are completely separated from the liquid, carefully remove the supernatant;
   2) Remove the EP tubes from the magnetic rack and add 40 µL of transposome diluent and 0.8 µL of 0.5 M MgCl₂ to each tube. Mix thoroughly by gently pipetting, and incubate in a 37°C incubator for 1 h.
10. Termination and decross-linking
   1) Add 5 µL 0.5 M EDTA, 4 µL 10% SDS, and 1 µL 20 mg/mL Proteinase K to each tube of sample incubated in the previous step;
   2) Transfer the sample to a PCR tube and incubate in a PCR instrument at 55°C for 2.5 h.

### S4. Recover the reaction termination products of CUT&Tag/ChiTag by the magnetic bead method:

1. DNA Extraction
1) Remove the terminated reaction product from the PCR instrument and gently pipette it evenly;
2) Vortex mix the Tagment DNA Exract Beads that have been equilibrated at room temperature for 10 min, add twice the volume of magnetic beads to the terminated reaction product, gently pipette to mix, and let stand at room temperature for 5 min;
3) Transfer the PCR tube to a magnetic rack and let stand for 5 min to allow the magnetic beads to separate from the liquid and carefully remove the supernatant;
4) Add 200 µL of freshly prepared 80% ethanol to the PCR tube. Let stand at room temperature for 1 min, then carefully remove the supernatant. Keep the PCR tube on the magnetic rack during this step;
5) Repeat step 4) once;
6) Place the PCR tube on a magnetic rack, open the PCR tube cap, and air-dry at room temperature (about 4 min) to allow the ethanol to evaporate completely.
7) Remove the PCR tubes from the magnetic rack, add 37 µL of TE Buffer to each tube, mix thoroughly by pipetting, and let stand at room temperature for 3 min;
8) Place the PCR tube on a magnetic rack and let stand for 2 min to completely separate the magnetic beads from the liquid. Carefully pipette 35 µL of the supernatant into a new sterile PCR tube. The sample can be stored at -20°C or proceed directly to the next PCR amplification step.

2. PCR Amplification
1) Preparation of PCR amplification system:

| Component | Volume (µL) |
|---|---|
| Extracted DNA | 35 |
| N5 Primer | 2.5 |
| N7 Primer | 2.5 |
| 5×AmpliMix | 10 |
| Total Volume | 50 |

2) Vortex mix, centrifuge quickly, and place in PCR instrument to start the cycle:

| | |
|---|---|
| 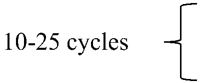 | 72 °C, 3 min; |
| | 98 °C, 30 sec; |
| | 98 °C, 15 sec; |
| | 60 °C, 10 sec; |
| | 72 °C, 8 sec; |
| | 72 °C, 2 min; |
| | 10 °C, ∞ |

3. PCR Product Purification
1) Take the NovoNGS DNA Clean Beads out of the 4°C refrigerator in advance, vortex mix thoroughly, and equilibrate at room temperature for 15 min;
2) Vortex mix the magnetic beads, add the magnetic beads to the PCR product at 1.3 times the volume thereof, pipette to mix, and let stand at room temperature for 5 min;
3) After centrifuging the PCR tube, place it on a magnetic rack and let stand for 5 min to completely separate the magnetic beads from the liquid, and carefully remove the supernatant;
4) Add 200 µL of freshly prepared 80% ethanol to wash the beads,let stand at room temperature for 1 min, then carefully remove the supernatant. Keep the PCR tube on the magnetic rack during this step;
5) Repeat step 4) once;
6) Place the PCR tube on a magnetic rack, open the PCR tube cap, and air-dry at room temperature (about 4 min) to allow the ethanol to evaporate completely;
7) Remove the PCR tubes from the magnetic rack, add 20 µL of TE Buffer to each tube, mix thoroughly by pipetting, and let stand at room temperature for 3 min;
8) Place the PCR tube on a magnetic rack and let stand for 2 min to completely separate the magnetic beads from the liquid, carefully pipette 20 µL of the supernatant into a new sterile PCR tube, the sample can be stored at -20°C.

### Example 1: Evaluation of the CUT&Tag assay using the classical CUT&Tag process on non-cross-linked, formaldehyde light cross-linked, and formaldehyde heavy cross-linked cells

### 1. Cell Preparation

**Table 2. Experimental group design I**

| Group | Formaldehyde Cross-linking Level | Primary Antibody |
|---|---|---|
| 1 (1-1/1-2) | Non-cross-linking | Pol II |
| 2 (2-1/2-2) | Light cross-linking | |
| 3 (3-1/3-2) | Heavy cross-linking | |
| 4 (4-1/4-2) | Non-cross-linking | CTCF |
| 5 (5-1/5-2) | Light cross-linking | |
| 6 (6-1/6-2) | Heavy cross-linking | |
| 7 (7-1/7-2) | Non-cross-linking | H3K4me3 |
| 8 (8-1/8-2) | Light cross-linking | |
| 9 (9-1/9-2) | Heavy cross-linking | |

1) Take 1.8 million K562 cells and divide them into 18 aliquots in 1.5 ml EP tubes, with 100,000 cells per sample, centrifuge at 600g for 5 min at room temperature and carefully remove the supernatant;
2) Add 1 mL of Wash Buffer to the cell pellets of groups 1/4/7 from the previous step, and add 1 mL of PBS to the cell pellets of groups 2/5/8 and 3/6/9. Mix gently by pipetting, then centrifuge at 600 g for 5 min at room temperature and discard the supernatant. Place the cells of groups 1/4/7 on ice, and proceed to the CUT&Tag assay after the cells of groups 2/5/8 and 3/6/9 have been processed;
3) Add 200 µL of the pre-prepared 0.1% formaldehyde-PBS solution to the cell pellets of groups 2/5/8 and react at room temperature for 2 min; add 200 µL of the pre-prepared 1% formaldehyde-PBS solution to the cell pellets of groups 3/6/9 and react at room temperature for 10 min;
4) Add 10.5 µL of 2.5 M glycine to the cells in groups 2/5/8, and 12.1 µL of 2.5 M glycine to the cells in groups 3/6/9 after finishing the cross-linking reaction. The reaction was incubated at room temperature for 5 min to terminate the cross-linking reaction;
5) Centrifuge at 1300 g for 5 min, discard the supernatant, add 1 mL of Wash Buffer to each of the cell pellets in groups 2/5/8 and 3/6/9, mix gently by pipetting, and centrifuge at 1300 g for 5 min, discard the supernatant;
6) Repeat steps 1-5) once;
7) Add 90 µL Wash Buffer to each sample to resuspend the cells.

### The following steps were performed using the same method as in the classical CUT&Tag experimental process described in S1:

2.Bind ConA magnetic beads to cells
3.Bind ConA magnetic beads-cells to primary antibodies
4.Bind ConA magnetic beads to cells
5.Bind ConA magnetic beads-cells to primary antibodies
6.Bind ConA magnetic beads-cells to secondary antibody and wash

### Recover the reaction termination products of CUT&Tag by the magnetic bead method described in S4.

Groups 1/2/3/4/5/6 were set to 17 cycles, and groups 7/8/9 were set to 15 cycles.

The PCR amplification products were analyzed by agarose gel electrophoresis to observe the bands, and the results are shown in Figure 2.

As shown in Figure 2, specific fragmentation bands were obtained for antibodies Pol II, CTCF, and H3K4me3 using the classic CUT&Tag process, confirming that these antibodies were functional. Comparison shows that fragmentation bands could still be obtained using the classic CUT&Tag process after formaldehyde light cross-linking. However, no fragmentation bands were obtained when the classic CUT&Tag process was applied to formaldehyde heavy cross-linked cells. Therefore, the classic CUT&Tag process is only suitable for formaldehyde light cross-linking, but not for formaldehyde heavy cross-linking.

### Example 2. Cells subjected to formaldehyde heavy cross-linking followed by classical CUT&Tag and ChiTag experimental process respectively

**Table 3. Experimental group design II**

| Group | Primary Antibody | experimental process |
|---|---|---|
| 1 (1-1/1-2) | Pol II | CUT&Tag |
| 2 (2-1/2-2) | H3K4me2 | |
| 3 (3-1/3-2) | CTCF | |
| 4 (4-1/4-2) | Pol II | ChiTag |
| 5 (5-1/5-2) | H3K4me2 | |
| 6 (6-1/6-2) | CTCF | |

1. Cell Preparation:
   1) Take 1.2 million K562 cells (100,000/group) in a new 1.5 mL centrifuge tube and centrifuge at 600 g for 5 min at room temperature, carefully remove the supernatant;
   2) Add 1 mL of PBS to the cell pellet from the previous step, mix gently by pipetting, centrifuge at 600 g for 5 min at room temperature, and discard the supernatant.
2. Formaldehyde cross-linking
   1) Prepare formaldehyde solution (formaldehyde-PBS) with a final concentration of 1% in advance;
   3) Add 400 µL of the pre-prepared 1% formaldehyde-PBS solution to the cell pellet from the previous step and react at room temperature for 10 min.
3. Termination of cross-linking
   1) Add 25 µL of 2.5 M glycine to the reaction system from the previous step and incubate at room temperature for 5 min to terminate the cross-linking reaction. During this time, divide the cells into 12 groups (1-1/1-2/2-1/2-1/3-1/3-2/4-1/4-2/5-1/5-2/6-1/6-2) with 35 µL/group;
   2) Centrifuge at room temperature for 5 minutes and discard the supernatant;
   3) Add 1 mL of Wash Buffer to each cell pellet, wash the cells, centrifuge at room temperature for 5 min, and discard the supernatant;
   4) Repeat steps 3-3) once;
   5) Resuspend the cell pellets in six groups of 1/2/3 with 90 µL Wash Buffer and proceed to the CUT&Tag process. Proceed cells in six groups of 4/5/6 to the ChiTag process.

### CUT&Tag experimental process (cells of groups 1/2/3):

**The following steps were performed using the same method as in the classic CUT&Tag experimental process described in S1:**
4.Bind ConA magnetic beads to cells
5.Bind ConA magnetic beads-cells to primary antibody
6.Bind ConA magnetic beads-cells to secondary antibody and wash 7. Transposome Binding and Washing
8. Fragmentation reaction

### ChiTag experimental process (cells of groups 4/5/6):

**The following steps were performed using the same method as in the ChiTag experimental process described in S3:**
4. Cell Permeabilization
5. Washing
6.Bind ConA magnetic beads to cells
7.Bind ConA-cells to primary antibody
8.Bind ConA-cells to secondary antibody and wash
9.Incubate ConA- cells with pA/G-Tn5-MgCl₂
10. Termination and decross-linking

**Recover the reaction termination products of CUT&Tag/ChiTag by the magnetic bead method.**

Groups Sets 1/3/4/6 were set to 19 cycles, and groups 2/5 were set to 18 cycles.

The PCR amplification products were observed by agarose gel electrophoresis, and the results are shown in Figure 3.

As shown in Figure 3, no fragmentation bands were obtained for antibodies Pol II, H3K4me2, and CTCF when the classic CUT&Tag process was applied to formaldehyde heavy cross-linked K562 cells. In contrast, distinct fragmentation bands were obtained using the ChiTag process of the present invention.

Performed qPCR quantitative analysis on the libraries, and the results are shown in Table 4. Combined with the results from Figure 3 and Table 4, it is evident that after formaldehyde heavy cross-linking of cells, the library yield constructed using the ChiTag process can be increased by 300 to 1000-fold compared to that constructed using the CUT&Tag process.

**Table 4. Library concentrations obtained using different experimental processs for different antibodies**

| Antibody | experimental processs | Library Concentration (nmol/L) |
|---|---|---|
| H3K4me2 | CUT&Tag | 0.052 |
| | ChiTag | 19.87 |
| Pol II | CUT&Tag | 0.031 |
| | ChiTag | 21.43 |
| CTCF | CUT&Tag | 0.01 |
| | ChiTag | 11.37 |

### Example 3 Detection of different antibodies in K562 cells using the classic CUT&Tag and ChiTag experimental process

**Table 5. Experimental group design III**

| Group | Primary Antibody | Secondary Antibody | Process |
|---|---|---|---|
| 1 (1-1/1-2) | H3K4me2 | Goat Anti-Rabbit | ChiTag |
| 2 (2-1/2-2) | Pol II | Goat Anti-Mouse | |
| 3 (3-1/3-2) | CTCF | Goat Anti-Rabbit | |
| 4 (4-1/4-2) | GR | Goat Anti-Mouse | |
| 5 (5-1/5-2) | H3K4me2 | Goat Anti-Rabbit | CUT&Tag |
| 6 (6-1/6-2) | Pol II | Goat Anti-Mouse | |
| 7 (7-1/7-2) | CTCF | Goat Anti-Rabbit | |
| 8 (8-1/8-2) | GR | Goat Anti-Mouse | |

1. Cell Preparation:
   Took 1.6 million K562 cells (100,000 cells/sample) into a new 1.5 mL centrifuge tube, divided equally into groups A and B, centrifuged at 600 g for 5 min, and removed the supernatant carefully. 1 mL of PBS was added to the cells in group A, and 1 mL of Wash Buffer was added to the cells in group B. The cells were divided equally into eight groups 5/6/7/8, mixed thoroughly by pipetting, and centrifuged at 600 g for 5 min. After discarding the supernatant, the cells in group A entered the ChiTag process, and the cells in group B were added with 90 µL of Wash Buffer to each group and entered the CUT&Tag process.

### ChiTag experimental process (cells of groups 1/2/3/4):

**The following steps were performed using the same method as in the classic ChiTag experimental process described in S3:**
2. Formaldehyde cross-linking
3. Termination of cross-linking
4. Cell Permeabilization
5. Washing
6. Bind ConA magnetic beads to cells
7. Bind ConA-cells to primary antibody
8. Bind ConA-cells to secondary antibody and wash
9. Incubate ConA- cells with pA/G-Tn5-MgCl₂
10. Termination and decross-linking

### CUT&Tag process (cells of groups 5/6/7/8)

**The following steps were performed using the same method as in the classic CUT&Tag experimental process described in S1:**
2. Bind ConA magnetic beads to cells
3. Bind ConA magnetic beads-cells to primary antibody
4. Bind ConA magnetic beads-cells to secondary antibody and wash
5. Transposome binding and washing
6. Fragmentation reaction

**Recover the reaction termination products of CUT&Tag/ChiTag by the magnetic bead method.**

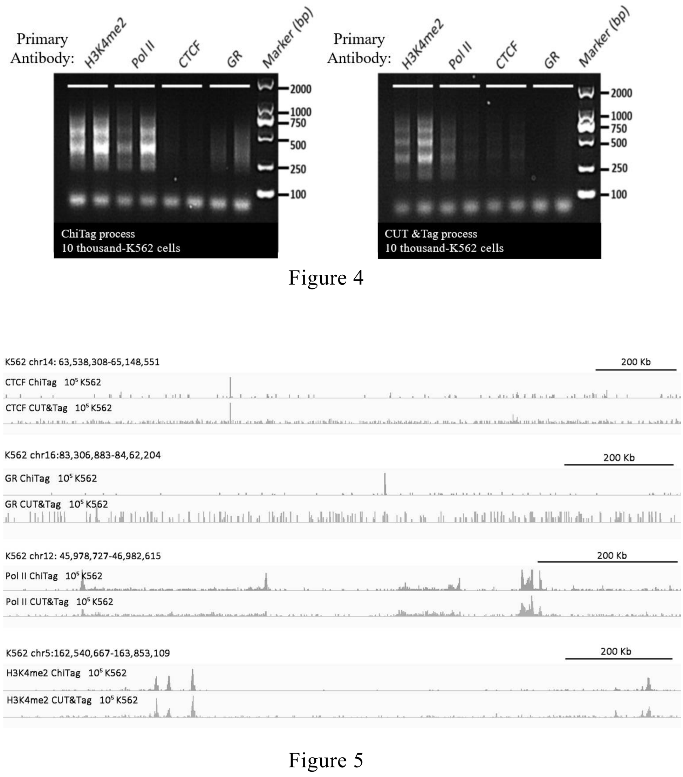

Groups 1/5 was set to 16 cycles, groups 2/3/6/7 were set to 18 cycles, and groups 4/8 was set to 22 cycles. PCR amplification products were detected by agarose gel electrophoresis, and the results are shown in Figure 4.

As shown in Figure 4, among the antibodies tested in this example, fragmentation bands were obtained for H3K4me2, Pol II, and CTCF using both the classic non-cross-linked CUT&Tag and the ChiTag process suitable for cross-linked cells. However, for the GR antibody, fragmentation bands were only obtained when using the ChiTag process. This result indicates that for some proteins with weaker DNA binding ability, the ChiTag process yields better fragmentation effect.

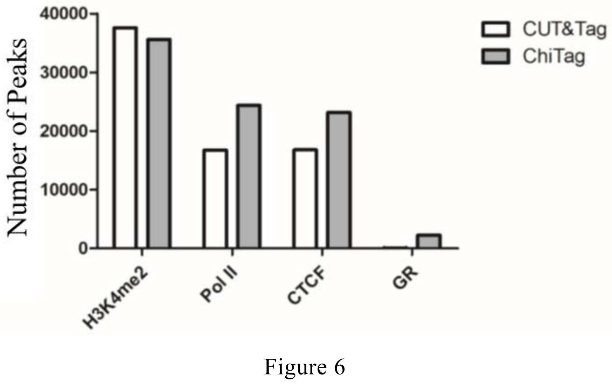

The PCR products were purified and sent to Novogene for sequencing. The sequencing results were analyzed on the Galaxy website (https://usegalaxy.org/), and the sequencing maps were viewed using IGV software. The sequencing results are shown in Figures 5, 6, and Table 6.

Figure 5 shows the signal-to-noise ratio maps (partially extracted) of different antibodies (CTCF, GR, Pol II, H3K4me2) in sequencing of libraries constructed using the CUT&Tag and ChiTag processes.

Observation of Figure 5 shows that within the given regions, compared to the classic CUT&Tag, ChiTag introduces formaldehyde cross-linking and it does not exhibit a significant decrease in signal-to-noise ratio. Notably, for the GR antibody, the fragmentation bands obtained using the CUT&Tag process consisted entirely of background noise, whereas the ChiTag process produced clear peaks with significant reads enrichment.

**Table 6. Comparison of sequencing data from libraries constructed using CUT&Tag and ChiTag process with different antibodies**

| Antibody name | H3K4me2 | | Pol II | | CTCF | | GR | |
|---|---|---|---|---|---|---|---|---|
| Library construction process | CUT&Tag | ChiTag | CUT&Tag | ChiTag | CUT&Tag | ChiTag | CUT&Tag | ChiTag |
| Library concentration (nmol/L) | 7.24 | 5 | 2.67 | 1.81 | 12.07 | 8.01 | 17.07 | 13.1 |
| Sequencing data size (GB) | 1.46 | 1.31 | 1.62 | 1.31 | 1.77 | 1.5 | 1.61 | 1.41 |
| Number of Peaks | 37573 | 35579 | 16743 | 24346 | 16777 | 23166 | 88 | 2252 |

Based on the data in Table 6 and Figure 6, it is evident that the CUT&Tag process shows a clear advantage only in detecting histone modifications. The H3K4me2 antibody yielded a higher number of peaks using the CUT&Tag process compared to ChiTag. In contrast, for the Pol II, CTCF, and GR antibodies, using the ChiTag process resulted in a greater number of peaks. This indicates a trend towards an improved signal-to-noise ratio for non-histone modification targets when using the ChiTag process, particularly for GR.

In summary, the ChiTag process demonstrates significant advantages in investigating protein-DNA interactions, especially weak protein-DNA interactions.

### Discussion:

Those skilled in the art are aware that CUT&Tag technology has limitations in many practical applications. For example, some transcription factors produce excellent results using ChIP-Seq technology, but have proven difficult when using CUT&Tag. For some transcription factors, while results can be obtained, the binding site signal intensity is weak and the background is high. Furthermore, CUT&Tag technology performs experiments in cells or nuclei. However, compositions in cells are complex, and cells contain numerous proteins and other substances. Without cross-linking, other biochemical reactions may occur during the 8 to10 hours experiment that could affect the results. Short-term light cross-linking with 0.1% to 0.2% formaldehyde may not be equivalent to long-term heavy cross-linking (1% to 5% formaldehyde, 10 to 60 min) for cell immobilization. Therefore, for detecting interactions involving DNA-binding proteins that exhibit dynamic binding to DNA, results from non-cross-linking or light cross-linking (0.1%-0.2% formaldehyde) conditions may not accurately reflect the true state of the experimental sample.

In conclusion, CUT&Tag technology faces significant challenges. A key step in ChIP-Seq technology is the use of 1% to 5% formaldehyde for heavy cross-linking, which fixes the binding state of DNA-binding proteins to chromatin, thereby maintaining their *in-situ* position throughout the experimental process and accurately reflecting the objective state of the sample at a specific time point. This is particularly important in genetic and developmental studies.

Faced with the shortcomings of CUT&Tag, researchers in the field often have to resort to the traditional and more complex ChIP technology. Therefore, resolving the technical difficulties of CUT&Tag to make it applicable to the study of most protein-DNA interactions, while retaining the simplicity and efficiency of CUT&Tag technology, is an urgent problem in the field of epigenetics research.

Classic CUT&Tag experiments use cells without formaldehyde cross-linking or with only light cross-linking using 0.1% to 0.2% formaldehyde for 2 to 3 min. During the experiment, there is a risk that the target protein might undergo other biochemical reactions in the complex environment of non-cross-linked cells. For some DNA-binding proteins with weak binding affinity or highly dynamic DNA binding, light cross-linking with 0.1% to 0.2% formaldehyde provides insufficient immobilization of the protein-DNA. Simply increasing the formaldehyde concentration (e.g., increasing to 0.5% to 3%) and prolonging the cross-linking time (5 to 60 min) within the CUT&Tag light cross-linking process easily leads to library construction failure or extremely low library yields, failing to meet sequencing requirements and severely reducing the sensitivity of the CUT&Tag technology.

The inventors of the present invention have unexpectedly discovered that the method combining formaldehyde heavy cross-linking for cell immobilization, followed by cell permeabilization treatment, with CUT&Tag technology (the ChiTag process of the present invention) surprisingly allows CUT&Tag technology to maintain its advantages of low cell requirement, high sequencing signal-to-noise ratio, and relative operational simplicity, while simultaneously incorporating the benefit of formaldehyde heavy cross-linking for cell immobilization from ChIP experiments. Furthermore, it dramatically increases the constructed library yield, thereby expanding the technology's applicability.

Additionally, after formaldehyde cross-linking immobilization, cell permeability decreases. Using traditional methods can easily lead to insufficient cell permeabilization. In the present invention, performing formaldehyde cross-linking immobilization first, followed by permeabilization treatment, allows subsequent antibodies, enzymes, etc., to enter the cells more easily. This results in higher antibody binding efficiency and more thorough fragmentation of enzyme. Consequently, the required number of cells can be reduced, meeting the needs of experiments with limited cell numbers. After the reaction, followed by decross-linking, purification, and amplification for library construction, it is easier to obtain a higher number of peaks and a higher signal-to-noise ratio.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

## Claims

1. A method for constructing a nucleic acid library, comprising:
(S1) providing a cell sample;
(S2) immobilizing the cell sample with a cross-linking agent having a final concentration of C1 (v/v) for a time period of t1 (min), where the immobilization integral I1 = C1 * t1, and I1 ≥ 2.5, to obtain a non-permeabilized cell sample;
(S3) permeabilizing the non-permeabilized cell sample to obtain a permeabilized cell sample;
(S4) washing the permeabilized cell sample to obtain a washed cell mixture;
(S5) mixing the cell mixture with pretreated magnetic beads to obtain a cell-magnetic bead complex;
(S6) treating the cell-magnetic bead conjugate with a primary antibody to obtain a primary antibody-cell-magnetic bead complex;
(S7) treating the primary antibody-cell-magnetic bead complex with a secondary antibody to obtain a secondary antibody-primary antibody-cell-magnetic bead complex;
(S8) treating the secondary antibody-primary antibody-cell-magnetic bead complex with a transposase to perform a fragmentation reaction to form DNA fragments;
(S9) terminating the reaction and decross-linking to obtain a reaction-terminated product; and
(S10) separating, purifying, and amplifying the DNA fragments in the reaction-terminated product to prepare a nucleic acid library.

2. The method of claim 1, wherein, in (S2), t1 is 5 to 60 min, preferably 10 to 20 min.

3. The method of claim 1, wherein, in (S2), the permeabilization agent is selected from one or more of the groups consisting of: Tween20, SDS, NP40 (or IGEPAL CA-630), Digtonin, CHAPS, sodium deoxycholate, or a combination thereof; preferably Tween20, SDS or NP40 (or IGEPAL CA-630).

4. The method of claim 1, wherein, in (S3), the permeabilization treatment comprises: treating the immobilized cell sample with a permeabilization buffer containing a permeabilization agent at a concentration C2 (v/v) for a time period of t2 (min) to obtain the permeabilized cell sample.

5. A kit for constructing a nucleic acid library using CUT&Tag technology, comprising:
(1) a cross-linking agent solution;
(2) a permeabilization buffer;
(3) a cell washing buffer;
(4) a fragmentation reaction reagent; and
(5) a decross-linking reagent.

6. The kit of claim 5, wherein, the permeabilization buffer further contains a cell protein protectant, which is 1 to 100 mM Glycine, 0.1%-2% (v/v) BSA, or other analogues.

7. The kit of claim 5, wherein, the permeabilization buffer contains a permeabilization agent selected from the group consisting of: Tween20, SDS, NP40, Digtonin, CHAPS, sodium deoxycholate, or a combination thereof; preferably Tween20, SDS or NP40.

8. The kit of claim 5, wherein, the concentration C2 of the permeabilization agent is 0.005% to 5%, preferably 0.01% to 2%.

9. A method for measuring protein-chromatin interactions, comprising:
(S1) constructing a nucleic acid library using the method of claim 1; and
(S2) sequencing the nucleic acid library to generate a plurality of sequencing reads for measuring protein-chromatin interactions.

10. The method of claim 9, wherein, the protein comprises a DNA-binding protein that has weak DNA binding affinity or highly dynamic DNA binding.
